# EUROPEAN PATENT APPLICATION

(11) **EP 1 782 727 A2**
(43) Date of publication of application: **09.05.2007**
(21) Application number: 07100474.1
(22) Date of filing: 03.10.2003
(51) Int. Cl.: A61B 1/12, A61M 16/04

(54) **Tube for inspecting internal organs of a body**

(30) Priority: 03.10.2002 US 415550 P
(62) Divisional of application: 03751231.6
(71) Applicant: Etview Ltd., 20179 Misgav (IL)
(72) Inventor: Gavrieli, Oren, 34618, Haifa (IL)
(74) Representative: Messulam, Alec Moses

(57) **Abstract**

A medical multifunctional tube (52) is described for inspecting internal organs of a body. The tube has a substantially free lumen for conveying various elements and also used for conveying liquids and gasses to and/or from organs (5) penetrated by said tube (52). The tube further comprising an imaging sensor (42) incorporated in the anterior face (22; 44) of said tube; at least one conduit of energy (54) to activate said imaging sensor (42) associated with the wall of said tube (52); at least one conducting element (28) for transmitting signals of said imaging sensor (42) to the rear end of said tube, at least one receiver for said signals; and cleaning means triggered by a timer or software for keeping said imaging sensor clear.

## Description

### TECHNICAL FIELD OF THE INVENTION

The present invention relates generally to a medical means of monitoring critically ill and anaesthetised patients including monitoring ventilated patients. More specifically, the invention is a device for monitoring patient's organs and cavities.

### BACKGROUND OF THE INVENTION

Insertion of tubes into patient's body organs, cavities and tracts is a common procedure in emergency and critical care medicine. An endotracheal tube may be inserted into the trachea of a patient who is in acute respiratory failure or is undergoing general anaesthesia. The endotracheal tube must be placed quickly and accurately and positioned with its tip in the mid portion of the patient's trachea to prevent accidental slipping and to provide proper seal and ventilation of both lungs. Similarly, a naso-gastric tube is commonly inserted through the nose or mouth into the stomach of patients who need artificial feeding or evacuation of the content of the stomach. Another tube that is frequently inserted into a body cavity during emergency treatment is a urinary catheter. This catheter is threaded through the urethra into the urinary bladder. The correct placement of these tubes and catheters throughout their use is critically important.

Many patients who are critically ill or undergoing general anaesthesia require artificial ventilation. For over 40 years the most common method of providing artificial ventilation has been by pumping compressed air into the patient's lungs through an endotracheal tube. This tube is inserted through the patient's mouth or nose and passed between the vocal cords into the trachea. Alternatively, a tube may be inserted into the trachea through a tracheotomy surgical incision.

For oral intubation the operator usually uses a laryngoscope, which consists of a handle and a blade. The operator inserts the blade into the patient's mouth and advances it until its tip lies in the pharynx beyond the root of the tongue. The handle is then used to manipulate the blade and push the tongue out of the way until the epiglottis and the vocal folds can be seen. The tip of the endotracheal tube can then be aimed and pushed between the vocal folds into the trachea. This method of insertion is used in the majority of intubations, but requires skill, training and experience and is only performed by specialized physicians and licensed paramedics.

An alternative method that is often used when difficult intubation is anticipated is over a fibre optic bronchoscope. First the bronchoscope is connected to a light source to provide the needed illumination of the field facing its tip. The shaft of the bronchoscope is then inserted through the endotracheal tube and moved in as far as possible. The tip of bronchoscope is then inserted into the patient's airway and advanced under visualization through the bronchoscope's eyepiece or a video display in between the vocal folds into the trachea. The endotracheal tube can now be pushed down the bronchoscope shaft and moved between the vocal folds into the trachea. The endotracheal tube can now be secured and the bronchoscope removed to free up the lumen of the endotracheal tube. While the bronchoscopic method is safer than with the laryngoscope, the equipment needed is expensive, delicate and more cumbersome and is seldom found in the field or on emergency medical vehicles.

Securing the endotracheal tube and preventing its inadvertent movement during use is critical to the prevention of dire accidents. Inflating a cuff that surrounds the tube near its tip occludes the space between the outer wall of the tube and the inner wall of the trachea to provide an airtight seal. The cuff is connected to the external end of the endotracheal tube through a thin channel in the tube's wall. The channel is connected to a one-way valve through which air can be injected to inflate the cuff to the desired pressure and volume. The cuff is also helpful in securing the tube in place, but additional fasteners are usually applied around the head to prevent the tube from slipping in or dislodging.

Once the tube has been inserted, it is mandatory to verify its correct position. Accidental insertion of the tube into the oesophagus or placing it too deep inside the airways, so that its tip is lodged in one of the main stem bronchi instead of in the trachea, may lead to catastrophic consequences and asphyxiation. Many methods are available to verify the endotracheal tube placement. Auscultation of both sides of the chest is usually done to verify symmetric air entry into both lungs. A chest x-ray is another well-tested method of verifying the tube placement. The x-ray picture reveals the relationships between the endotracheal tube tip and the tracheal first bifurcation (carina). X-ray pictures may be and should be taken whenever an endotracheal tube is placed or repositioned. Additionally, the tube placement may be verified through a fibre optic bronchoscope, by a suction bulb, or through sending and receiving an acoustic signal. These methods are used to verify the initial placement of the endotracheal tube. There are no currently available means for continuous monitoring of the actual placement of the tube.

The advantages of fibre optic visualization were combined with the simple design of the laryngoscope as disclosed by several patents and scientific papers. Additionally, the use of visualization stylets which include means for seeing the airways during the insertion of an endotracheal tube have been described. However, there are no known methods for incorporating the visualization means permanently into the anterior face of the endotracheal tube so that visualization of the airways can be accomplished during the insertion and continuously thereafter.

### SUMMARY OF THE INVENTION

The present provides a multifunction tube as hereinafter set forth in Claim 1 of the appended claims.

### BRIEF DESCRIPTION OF THE DRAWINGS

Fig. 1 is a schematic isometric scheme of the tube of the invention incorporating three types of conduits;
Fig. 2 is a schematic isometric description of a portion of the anterior face of the tube of the invention into which a miniature video camera is incorporated;
Fig. 3 is a schematic description of the items commuting along the tube of the invention, related to the performance of inspection tasks.

### DETAILED DESCRIPTION OF THE PRESENT INVENTION

In the illustrated embodiment of the invention, a multifunctional inspection tube is provided for collecting information about internal cavities and spaces in the body of a patient or an animal in association with the insertion of an inspection tube in the body. The multifunctional inspection tube is a modified medical tube such as an endotracheal tube, catheter, a gastric feeding tube. In accordance with the present invention the tube is equipped with means to examine both the positioning of the inspection tube with respect to body organs and the functional aspects of the body during and after the insertion. Thus, the tube of the invention may be used to perform not only customary medical treatment tasks of conveying gasses and or liquids to and from the penetrated organs, but also inspection tasks that examine the reaction to such treatment and otherwise the condition of the penetrated organs.

The multifunctional inspection tube of the invention incorporates a means of receiving signals relating to the condition of the penetrated organs such as visual and audio signals by employing suitable sensors incorporated at or near the anterior face of a tube. The signals produced by the sensors are transmitted via wires or communication fibres running along the length of the tube to a connector or a wireless transmitter located at the posterior portion of the tube near its standard connector to the ventilation source, gastric tube feeder or urinary collecting device. The signals are received by a receiver containing a suitable signal conditioning means for subsequent processing, display, recording and/or monitoring.

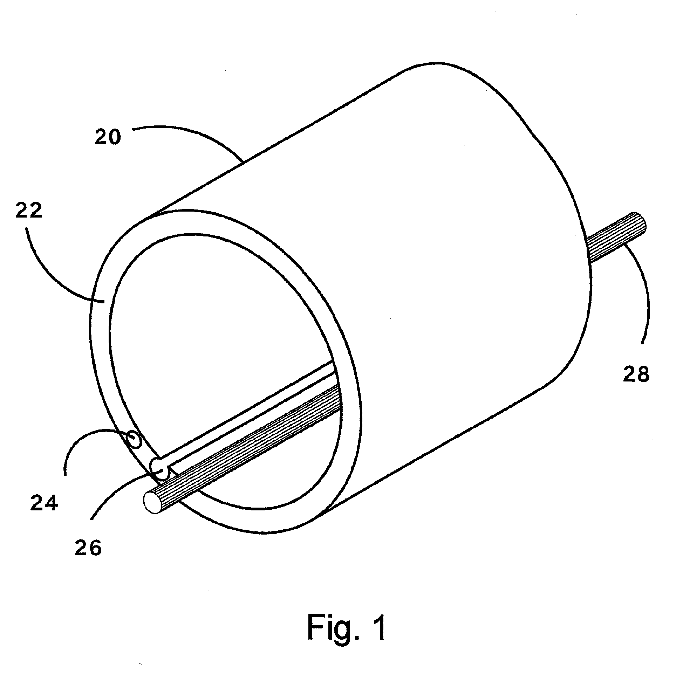

The structural concept of the invention is better explained with reference to Fig. 1. A portion 20 of a multifunctional inspection tube of the invention is shown, including an anterior face 22. Channels and a conductor are associated with the wall of the tube. A totally embedded channel 24 runs along the length of the inspection tube within its wall, alongside an open recessed channel 26. A conducting element 28 runs along the length of the tube without being embedded in the wall of the tube, rather it is attached to the wall of the tube and occupies a space in the lumen of the tube. The conduit may be partially embedded in the tube or it may be inserted within a recess or it may be threaded within a totally embedded channel without being attached to the tube. Even in embodiments in which the lumen contains a conducting element attached to the wall as described above, the lumen of the tube is still largely free for transferring liquids or gasses in both directions. With this respect, embodiment in which the channels and conducting elements embedded or wholly inserted in the wall may be preferable.

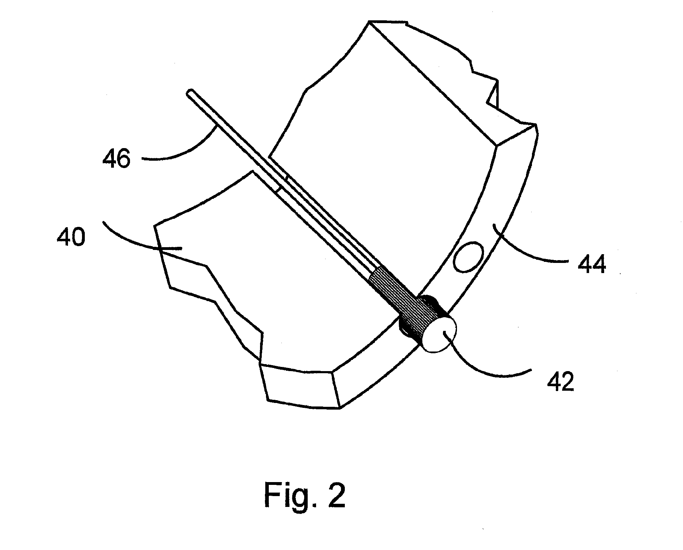

A typical feature of the multifunctional inspection tube is the acquisition of internal images of the body. For acquiring the images, an image sensor may be employed, such as a miniature electronic camera employing a CCD or a CMOS chip incorporated in the anterior face of the inspection tube. In one embodiment, the camera is incorporated in a recess in the wall of the tube as described in Fig. 2 to which reference is now made. A portion of an inspection tube is shown, within the inner side 40 of which, a camera 42 is inserted in a recessed channel, protruding from the anterior face 44 of the tube. The signal of the camera is transmitted by a conducting element 46, typically a copper wire or an optical fibre. The camera's lens is facing away from the tube. The signals arriving from the camera are fed to a receiver and may be subsequently displayed on a screen, which may be a stand-alone mini-screen, an ordinary video screen, or a portion of the display screen ordinarily used to monitor the physiological parameters and well-being of the patient.

In some embodiments of the invention, a fibre optical element running along the length of the tube is used to convey light to illuminate the field of view ahead of the anterior face of the inspection tube. Alternatively, a light source may be associated with the proximal face of the tube. Examples for light sources are miniature halogen lamps, light emitting diodes (LED), lasers, or any other kind of light-emitting source of suitable size. An alternative method of illumination is by constructing the inspection tube made of light-conducting material.

In the invention, means are employed for keeping the lens of the camera and/or other sensors clean and clear. The airways, stomach or urinary bladder of an ill patient are often filled with secretions that may be thick and viscous. Thus, it is quite possible that the secretions may lodge on the lens and obscure its field of view, or on other sensors thereby modifying their responsiveness. To overcome such an obstacle, a constant or intermittent flow of air or physiological fluid is pumped through a channel in the tube's wall, whereby the outlet of the channel is aimed directly over and around the lens or the sensor's active surface. This flow is triggered to emit flow upon command from a timer or a software program that monitors the signal and determines when clearing action is required.

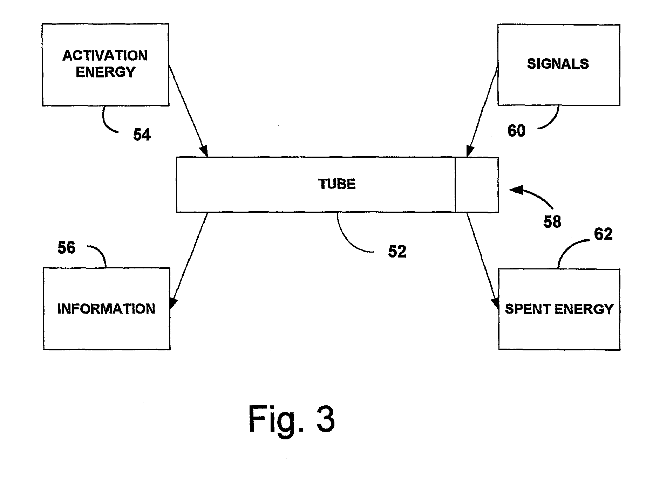

In general, the inspection tube is used as bidirectional conveying platform for various elements required for the fulfilment of its inspection tasks. This is described schematically in Fig. 3 to which reference is now made. Tube 52 receives activation energy 54 of one or several types on its rear end, and downloads information 56, raw or processed at the same end. At the anterior and 58, the tube receives signals 60 of one or several types, and spends energy 62 as will be elaborated later on.

In some embodiments of the invention, a microphone is employed in the tube. Such a microphone can be incorporated in the wall of the tube. Such a microphone receives acoustic signals from at least the vicinity of the tube's anterior, and transfers the signals, raw or processed to the rear of the tube for further downloading and processing.

A plurality of sensors can be effectively employed in the anterior face of the tube of the invention, the non-exhaustive list includes cameras, video cameras, microphones, pressure transducers and thermal sensors. Gas sensors, for example sensors for particular gasses such as oxygen and carbon dioxide may also be employed. The energy required to activate such sensors is supplied by conduits of energy such as electric wires incorporated in the tube. In addition, auxiliary energy can be supplied to the vicinity of the anterior face of the tube for the purpose of cleaning and clearing the sensors active facets by flushing them with cleaning media such as gases, humidified air or oxygen, or liquids, typically a physiological solution, through channels in the wall of the tube. Liquids and or gases for flushing are energized and conducted typically via a totally embedded channel.

The inspection tube of the invention may be used alone or in combination with other catheters and tubes that are ordinarily inserted into a body organ, tract or cavity such as the oesophagus, the stomach, the intestine, the colon, the urinary bladder, the pleural space, lung airways and/or the peritoneal cavity. The present technology may be applied in various medical practices and treatments such as: artificial ventilation of the lung, feeding or removing the content of the stomach, draining urine from the bladder, draining the gas and faeces from the colon, and draining or injecting into a surgically accessed cavity such as the pleural space, or the peritoneal cavity.

The sensors of the tube transmit one or more signal types, which are either pre-processed in the sensor for example on the CCD chip, or may be sent raw, to be further processed by analogue or digital circuits to yield information relating to the status of the organ or body cavity inspected. The receiving and or processing devices such as, monitors, displays, storage means, analysers, DSP processors, computers and generators of alarm signals are typically connected by one or a plurality of connectors to the tube. The tube of the invention may be used for insertion through orifices such as the nose, mouth, urethral meatus, rectum, or a surgical incision.

The transmission of raw or pre-processed signals is affected through conductors along the tube such as wires or optical fibres, which connect to a connector at the rear of the tube. A wireless transmitter or transceiver may be applied anywhere suitable on the tube, typically at the rear, for communicating with a console containing a receiver and processor and or a control module.

The inspection tube of the invention may also be used to detect changes in indications of vital functions of a patient. Accordingly, image and acoustic signal are being detected, processed and compared to a reference base picture or sound structure. An alarm is set as soon as certain changes in the indication pass a predetermined threshold. For example, the accumulation of secretions, or development of excessive or diminished lung noises are abnormal.

## Claims

1. A medical multifunctional tube (52) for inspecting internal organs of a body, the tube having a substantially free lumen for conveying various elements and at least one item selected from the group consisting of gasses and liquids into and out of organs (5) penetrated by said tube (52), said tube further comprising:
an imaging sensor (42) incorporated in the anterior face (22; 44) of said tube;
at least one conduit of energy (54) to activate said imaging sensor (42) associated with the wall of said tube (52);
at least one conducting element (28) for transmitting signals of said imaging sensor (42) to the rear end of said tube,
at least one receiver for said signals; and
cleaning means triggered by a timer or software for keeping said imaging sensor clear.

2. A multifunctional tube (52) as claimed in claim 1 and wherein the wall of said tube (52) comprises a channel (24, 26).

3. A multifunctional tube (52) as claimed in claim 2 and wherein the wall (20) of said tube (52) comprises a totally embedded channel (24).

4. A multifunctional tube (52) as claimed in claim 2 and wherein the wall of said tube comprises a recessed channel (26).

5. A multifunctional tube (52) as claimed in claim 1 and wherein a lighting element is associated with said anterior face (44) of said tube.

6. A multifunctional tube (52) as claimed in claim 5 and wherein said lighting element is an optical fibre running along the wall of said tube.

7. A multifunctional tube (52) as claimed in claim 5 and wherein said lighting element is a light emitting source.

8. A system comprising the multifunctional tube (52) of any preceding claim, comprising at least one circuit for processing signals provided by said sensor (42) so as to yield information relating to the status of the organ or body cavity being inspected by said tube (52).

9. A system as claimed in claim 8, further comprising an alarm to be triggered upon a change of an indication of vital functions of the patient according to at least one signal from said sensor (42).

10. A system as claimed in claims 8 or 9, further comprising an audio sensor for being connected to said at least one circuit for processing signals provided by said sensor (42).

11. A system as claimed in any of claims 8 to 10, wherein said at least one circuit communicates with said sensor or sensors through wired or wireless communication.

12. A multifunctional tube as claimed in any of claims 1 to 7, wherein the sensor (42) is adapted to send signals to display and monitoring devices so as to yield information relating to the status of the organ or body cavity being inspected by said tube (52).

13. A multifunction tube (52) as claimed in any preceding claim, further including a microphone for receiving acoustic signals from the vicinity of the anterior of the tube.
